# EUROPEAN PATENT APPLICATION

(11) **EP 4 417 200 A1**
(43) Date of publication of application: **21.08.2024**
(21) Application number: 22881017.2
(22) Date of filing: 11.10.2022
(51) Int. Cl.: A61K 31/202, A23D 9/00, A23L 33/115, A61K 31/232, A61P 17/00, A61P 17/06, A61P 17/08, A61P 17/10, A61P 17/16

(54) **AGENT FOR PREVENTING, TREATING, OR IMPROVING INFLAMMATORY SKIN DISEASE**

(30) Priority: 13.10.2021 JP 2021168413
(71) Applicant: Kao Corporation, Tokyo 103-8210 (JP)
(72) Inventor: SAYAMA, Keimon, Tokyo 113-8421 (JP); FUKAGAWA, Satoko, Tokyo 113-8421 (JP); ISHIKAWA, Junko, Tokyo 113-8421 (JP); SASAKI, Aya, Tokyo 131-8501 (JP)
(74) Representative: Vossius & Partner Patentanwälte Rechtsanwälte mbB
(86) International application number: PCT/JP2022/037897
(87) International publication number: WO 2023/063322

(57) **Abstract**

Provided is an agent for preventing, treating, or improving inflammatory skin disease. An agent for preventing, treating, or improving inflammatory skin disease comprising, as an active ingredient, a fat or oil in which a content of α-linolenic acid in fatty acids constituting the fat or oil is 40 mass% or more, and a content of diacylglycerol is 25 mass% or more.

## Description

### Field of the Invention

The present invention relates to an agent for preventing, treating, or improving inflammatory skin diseases such as atopic dermatitis.

### Background of the Invention

Atopic dermatitis (AD) is one of skin diseases associated with inflammation and is caused by genetic and environmental factors. Recent studies have revealed that both immunological abnormalities and skin barrier dysfunction are involved in the pathogenesis of atopic dermatitis. The barrier function of the skin is mainly carried out by the stratum corneum and the sebum barrier which covers its surface, and their normal function prevents water loss from the body and the entry of various external stimuli and substances. However, in atopic dry skin, which is often seen in patients with atopic dermatitis, the skin barrier function is broken down, and the entry of environmental allergens and stimuli, the onset and aggravation of eczema, and the induction of pruritus act in a vicious cycle, causing the condition to become chronic and intractable. Abnormal skin barrier function is also evident in patients with psoriasis vulgaris and other similar skin conditions. Therefore, when treating atopic dermatitis and the like, it is important to bring the symptoms into a state of remission by suppressing inflammation and normalizing the skin barrier function.

α-linolenic acid (C18:3, ALA) is an ω-3 highly unsaturated fatty acid contained in high amounts in linseed oil and perilla seed oil (shiso seed oil). **α-**linolenic acid has been reported to have anti-atherosclerotic, hypertensive, and anti-allergic properties. It has also been reported that ingestion of linseed oil improves transepidermal water loss (TEWL) values in dry and sensitive skin (Non Patent Literature 1) .

On the other hand, fats or oils containing high concentrations of diacylglycerols have been reported to have physiological effects such as suppressing postprandial increases in blood triglycerides (neutral fat) and having little accumulation in the body. For example, Patent Literature 1 reports that fat or oil compositions containing 60 to 100% by weight of diglycerides, in which 15 to 90% by weight of the constituent fatty acids are **ω**-3 unsaturated fatty acids having less than 20 carbon atoms and the weight ratio of cis-**ω**-3 unsaturated fatty acids/(cis-**ω**-6 unsaturated fatty acids + saturated fatty acids + trans-unsaturated fatty acids) is 1 to 6, have excellent visceral fat-burning properties, body fat-burning properties, and the like.

However, it is not known that diacylglycerol-containing fats or oils, which contain high amounts of **α-**linolenic acid as a constituent fatty acid, are useful in the prevention or treatment of inflammatory skin diseases such as atopic dermatitis.

(Patent Literature 1) JP-A-2002-138296

(Non Patent Literature 1) Jean-Marc Maurette. OCL. 2008, 15(4), 257-261

### Summary of the Invention

The present invention relates to the following 1) to 12) :
1) An agent for preventing, treating, or improving inflammatory skin disease comprising, as an active ingredient, a fat or oil in which a content of **α-**linolenic acid in fatty acids constituting the fat or oil is 40 mass% or more, and a content of diacylglycerol is 25 mass% or more.
2) An agent for improving skin barrier function comprising, as an active ingredient, a fat or oil in which a content of **α**-linolenic acid in fatty acids constituting the fat or oil is 40 mass% or more, and a content of diacylglycerol is 25 mass% or more.
3) A food for preventing or improving inflammatory skin disease comprising, as an active ingredient, a fat or oil in which a content of **α**-linolenic acid in fatty acids constituting the fat or oil is 40 mass% or more, and a content of diacylglycerol is 25 mass% or more.
4) A food for improving skin barrier function comprising, as an active ingredient, a fat or oil in which a content of **α**-linolenic acid in fatty acids constituting the fat or oil is 40 mass% or more, and a content of diacylglycerol is 25 mass% or more.
5)Use of a fat or oil for producing an agent for preventing, treating, or improving inflammatory skin disease, in which a content of **α**-linolenic acid in fatty acids constituting the fat or oil is 40 mass% or more, and a content of diacylglycerol is 25 mass% or more.
6) A fat or oil for use in preventing, treating, or improving inflammatory skin disease, in which a content of **α**-linolenic acid in fatty acids constituting the fat or oil is 40 mass% or more, and a content of diacylglycerol is 25 mass% or more.
7) Non-therapeutic use of a fat or oil for preventing, treating, or improving inflammatory skin disease, in which a content of **α**-linolenic acid in fatty acids constituting the fat or oil is 40 mass% or more, and a content of diacylglycerol is 25 mass% or more.
8) Use of a fat or oil for producing an agent for improving skin barrier function, in which a content of **α-**linolenic acid in fatty acids constituting the fat or oil is 40 mass% or more, and a content of diacylglycerol is 25 mass% or more.
9) A fat or oil for use in improving skin barrier function, in which a content of **α**-linolenic acid in fatty acids constituting the fat or oil is 40 mass% or more, and a content of diacylglycerol is 25 mass% or more.
10) Non-therapeutic use of a fat or oil for improving skin barrier function, in which a content of **α**-linolenic acid in fatty acids constituting the fat or oil is 40 mass% or more, and a content of diacylglycerol is 25 mass% or more.
11) A method for preventing, treating, or improving inflammatory skin disease, comprising administering or applying, to a subject, a fat or oil in which a content of **α**-linolenic acid in fatty acids constituting the fat or oil is 40 mass% or more, and a content of diacylglycerol is 25 mass% or more.
12) A method for improving skin barrier function, comprising administering or applying, to a subject, a fat or oil in which a content of **α**-linolenic acid in fatty acids constituting the fat or oil is 40 mass% or more, and a content of diacylglycerol is 25 mass% or more.

### Brief Description of Drawings

[Figure 1] Graph showing changes in dermatitis score (AD) .
[Figure 2] Graph showing changes in auricular thickening.
[Figure 3] Graph showing changes in transepidermal water loss (TEWL).
[Figure 4] Graph showing the results of analyzing cytokine expression at the site of induced dermatitis.

### Detailed Description of the Invention

The present invention relates to providing an agent for preventing, treating, or improving inflammatory skin diseases.

The present inventors evaluated the efficacy of a diacylglycerol-containing fat or oil with a high **α-**linolenic acid content using atopic dermatitis model mice, and as a result, found that the dermatitis score and auricular thickening were suppressed, the increase in transepidermal water loss (TEWL) was suppressed, and the increase in various cytokines related to inflammatory skin diseases was suppressed in mice raised on feed supplemented with a diacylglycerol-containing fat or oil with a high α-linolenic acid content, indicating that the fat or oil was useful in the prevention or treatment of inflammatory skin diseases such as atopic dermatitis.

The present invention provides a medicament, food, and the like effective in preventing, treating, or improving inflammatory skin diseases by suppressing dermatitis and improving skin barrier function.

In the present invention, "fat or oil" refers to ester compounds of fatty acids and glycerol, and includes one or more of monoacylglycerol, diacylglycerol and triacylglycerol. The type of fat or oil is not particularly limited as long as it can be used as an edible fat or oil.

In the fat or oil used in the present invention, the content of α-linolenic acid in the fatty acids constituting the fat or oil is 40 mass% or more, and the content of diacylglycerol is 25 mass% or more. Hereinafter, the "fat or oil in which the content of α-linolenic acid in the fatty acids constituting the fat or oil is 40 mass% or more, and the content of diacylglycerol is 25 mass% or more" may be simply referred to as the "fat or oil of the present invention" in the present specification.

The content of **α**-linolenic acid in the fatty acids constituting the fat or oil of the present invention is 40 mass% or more, and from the viewpoint of physiological efficacy, it is preferably 45 mass% or more, more preferably 50 mass% or more, further more preferably 52 mass% or more, and from the viewpoint of oxidative stability, preferably 80 mass% or less, more preferably 70 mass% or less, further more preferably 60 mass% or less.

The content of **α**-linolenic acid in the fatty acids constituting the fat or oil is 40 mass% or more and 80 mass% or less, preferably 45 mass% or more and 80 mass% or less, more preferably 50 mass% or more and 80 mass% or less, more preferably 50 mass% or more and 70 mass% or less, further more preferably 52 mass% or more and 70 mass% or less, further more preferably 52 mass% or more and 60 mass% or less. The amount of fatty acid in the present specification is indicated in terms of free fatty acids.

In the present invention, the constituent fatty acids other than **α**-linolenic acid constituting the fat or oil are not particularly limited and may be either saturated fatty acids or unsaturated fatty acids.

From the viewpoint of flavor and industrial productivity of the fat or oil, the content of unsaturated fatty acids in the fatty acids constituting the fat or oil is preferably 60 mass% or more and 100 mass% or less, more preferably 70 mass% or more and 100 mass% or less, further more preferably 75 mass% or more and 99 mass% or less, further more preferably 80 mass% or more and 98 mass% or less. The number of carbon atoms in the unsaturated fatty acid is preferably from 14 to 24, more preferably from 16 to 22 from the viewpoint of physiological efficacy.

The content of linoleic acid (C18:2) in the fatty acids constituting the fat or oil is preferably 5 mass% or more, more preferably 10 mass% or more from the viewpoint of industrial productivity, and preferably 40 mass% or less, more preferably 30 mass% or less, further more preferably 20 mass% or less from the viewpoint of physiological efficacy.

The content of linoleic acid (C18:2) in the fatty acids constituting the fat or oil is preferably 5 mass% or more and 40 mass% or less, more preferably 10 mass% or more and 30 mass% or less, further more preferably 10 mass% or more and 20 mass% or less.

The content of oleic acid (C18:1) in the fatty acids constituting the fat or oil is preferably 10 mass% or more from the viewpoint of industrial productivity, and preferably 50 mass% or less, more preferably 40 mass% or less, further more preferably 30 mass% or less from the viewpoint of physiological efficacy.

The content of oleic acid (C18:1) in the fatty acids constituting the fat or oil is preferably 10 mass% or more and 50 mass% or less, more preferably 10 mass% or more and 40 mass% or less, further more preferably 10 mass% or more and 30 mass% or less.

The total content of saturated fatty acids in the fatty acids constituting the fat or oil is preferably 6.0 mass% or less, more preferably 5.5 mass% or less, further more preferably 5.0 mass% or less from the viewpoint of appearance, physiological efficacy, and industrial productivity of the fat or oil. From the viewpoint of industrial productivity, it is preferably 0.5 mass% or more.

The total content of saturated fatty acids in the fatty acids constituting the fat or oil is preferably 0.5 mass% or more and 6.0 mass% or less, further more preferably 0.5 mass% or more and 5.5 mass% or less, further more preferably 0.5 mass% or more and 5.0 mass% or less.

The type of saturated fatty acid is not particularly limited, but saturated fatty acids having from 14 to 24 carbon atoms are preferred, saturated fatty acids having from 16 to 22 carbon atoms are more preferred, and saturated fatty acids having 16, 18, and 20 carbon atoms are further more preferred.

The content of diacylglycerol in the fat or oil of the present invention is 25 mass% or more, and from the viewpoint of effective exhibition of the effect and physiological efficacy, it is preferably 30 mass% or more, more preferably 50 mass% or more, further more preferably 55 mass% or more, further more preferably 60 mass% or more, further more preferably 65 mass% or more, further more preferably 70 mass% or more, further more preferably 80 mass% or more, further more preferably 83 mass% or more, further more preferably 84 mass% or more, and preferably 96 mass% or less, more preferably 95 mass% or less, further more preferably 94 mass% or less.

The content of diacylglycerol in the fat or oil is preferably 25 mass% or more and 96 mass% or less, more preferably 30 mass% or more and 96 mass% or less, further more preferably 50 mass% or more and 96 mass% or less, further more preferably 50 mass% or more and 95 mass% or less, further more preferably 55 mass% or more and 95 mass% or less, further more preferably 60 mass% or more and 95 mass% or less, further more preferably 65 mass% or more and 95 mass% or less, further more preferably 65 mass% or more and 94 mass% or less, further more preferably 70 mass% or more and 94 mass% or less, further more preferably 80 mass% or more and 94 mass% or less, further more preferably 83 mass% or more and 94 mass% or less, further more preferably 84 mass% or more and 94 mass% or less.

In the present invention, the fatty acids constituting diacylglycerol may be either saturated fatty acids or unsaturated fatty acids, and the content of **α-**linolenic acid in the fatty acids constituting diacylglycerol is preferably 40 mass% or more from the viewpoint of effectively exhibiting the effect.

From the same viewpoint, the content of **α**-linolenic acid in the fatty acids constituting diacylglycerol is preferably 45 mass% or more, more preferably 50 mass% or more, further more preferably 52 mass% or more, and from the viewpoint of oxidative stability, preferably 80 mass% or less, more preferably 70 mass% or less, further more preferably 60 mass% or less.

The content of **α**-linolenic acid in the fatty acids constituting diacylglycerol is 40 mass% or more and 80 mass% or less, preferably 45 mass% or more and 80 mass% or less, more preferably 50 mass% or more and 80 mass% or less, more preferably 50 mass% or more and 70 mass% or less, further more preferably 52 mass% or more and 70 mass% or less, further more preferably 52 mass% or more and 60 mass% or less.

The fat or oil of the present invention may contain triacylglycerol, the content of which is, from the viewpoint of industrial productivity of the fat or oil, preferably 1 mass% or more, more preferably 2 mass% or more, further more preferably 5 mass% or more, and preferably 75 mass% or less, more preferably 72 mass% or less, more preferably 50 mass% or less, more preferably 25 mass% or less.

The content of triacylglycerol in the fat or oil is preferably 1 mass% or more and 75 mass% or less, more preferably 2 mass% or more and 75 mass% or less, further more preferably 2 mass% or more and 72 mass% or less, further more preferably 5 mass% or more and 72 mass% or less, further more preferably 5 mass% or more and 50 mass% or less, further more preferably 5 mass% or more and 25 mass% or less.

From the viewpoint of flavor and industrial productivity of the fat or oil, the content of monoacylglycerol in the fat or oil is preferably 3 mass% or less, more preferably 2 mass% or less, further more preferably 1.51 mass% or less, and preferably more than 0 mass%. The content of monoacylglycerol in the fat or oil may be 0 mass%.

The fatty acid composition of triacylglycerol, diacylglycerol and monoacylglycerol is preferably the same from the viewpoint of industrial productivity of the fat or oil.

The fat or oil of the present invention may contain free fatty acids or salts thereof as impurities. The content of free fatty acids or salts thereof in the fat or oil is, from the viewpoint of flavor, preferably 3 mass% or less, more preferably 2 mass% or less, further more preferably 1 mass% or less, and preferably more than 0 mass%. The content of free fatty acids or salts thereof in the fat or oil may be 0 mass%.

The fat or oil of the present invention can be obtained by an esterification reaction between fatty acids derived from fats or oils and glycerol, a transesterification reaction between fats or oils and glycerol (glycerolysis), or the like in accordance with conventional methods. Conventional edible fats or oils may be mixed if necessary.

Esterification and glycerolysis reactions are roughly classified into chemical methods using chemical catalysts such as alkali metals or their alloys, oxides or hydroxides of alkali metals or alkaline earth metals, and alkoxides of alkali metals or alkaline earth metals, and enzymatic methods using enzymes such as lipases.

Of these, an esterification reaction between fatty acids obtained by hydrolyzing the fats or oils as described below (fractionated fatty acids) and glycerol is preferred from the viewpoint of controlling fatty acid composition.

In the present invention, the origin of the fats or oils is not particularly limited as long as they can be used as an edible fat or oil, and may be either from plants or animals. Example thereof include plant-derived fats or oils such as soybean oil, rapeseed oil, safflower oil, rice oil, corn oil, sunflower oil, cottonseed oil, olive oil, sesame oil, peanut oil, coix lacryma-jobi seed oil, wheat germ oil, perilla oil (shiso oil), linseed oil, perilla oil (egoma oil), chia seed oil, sacha inchi oil, walnut oil, kiwi seed oil, salvia seed oil, grape seed oil, macadamia nut oil, hazelnut oil, pumpkin seed oil, camellia oil, tea seed oil, borage oil, palm oil, palm olein, palm stearin, coconut oil, palm kernel oil, cacao butter, sal fat, shea butter, and algal oil; animal fats or oils such as fish oil, seal oil, lard, beef tallow, and butterfat; and transesterified oils thereof, hydrogenated oils thereof, fractionated oils thereof and the like.

These oils may be used alone, or in combination as appropriate. Of these, plant-derived fats or oils are preferably used from the viewpoint of usability, liquid fats or oils with excellent low-temperature resistance are further more preferably used, and one or more fats or oils selected from the group consisting of perilla oil (shiso oil), linseed oil, and perilla oil (egoma oil) are further more preferably used as they are rich in **α-**linolenic acid. Liquid fats or oils refer to fats or oils which are liquid at 20°C when subjected to a cooling test in accordance with the Standard Methods for the Analysis of Fats, Oils, and Related Materials 2.3.8-27. The edible fats or oils are preferably purified fats or oils which have undergone a purification step.

The fatty acids derived from the fats or oils can be obtained by hydrolyzing the fats or oils. Examples of methods for hydrolyzing the fats or oils include high temperature-pressure cleavage and enzymatic cleavage. High temperature-pressure cleavage is a method of adding water to the fat or oil and reacting them under high temperature and pressure conditions to obtain the fatty acids and glycerol. Enzymatic cleavage is a method of adding water to the fat or oil and reacting them under low temperature conditions using a fat or oil hydrolase to obtain fatty acids and glycerol.

The hydrolysis reaction can be performed in accordance with a conventional method.

After hydrolysis of the fat or oil, the hydrolysates are preferably fractionated to remove any solids. Examples of fractionation methods include solvent fractionation, natural fractionation (dry fractionation), and wet fractionation.

Examples of the means for removing precipitated solids include static separation, filtration, centrifugation, and separation by mixing the fatty acids with an aqueous solution of a wetting agent.

The esterification reaction between the fatty acids derived from the fat or oil and glycerol is preferably performed under mild conditions by an enzymatic method, as it is superior from the viewpoint of flavor and the like.

The amount of enzyme used can be determined as appropriate considering the activity of the enzyme, but from the viewpoint of improving the reaction rate, it is preferably from 1 to 30 mass%, more preferably from 2 to 20 mass% of the total mass of raw materials for the esterification reaction when an immobilized enzyme is used.

The reaction temperature of the esterification reaction is preferably from 0 to 100°C, more preferably from 20 to 80°C, and further more preferably from 30 to 60°C, from the viewpoint of improving the reaction rate and suppressing enzyme deactivation. The reaction time is preferably within 15 hours, more preferably from 1 to 12 hours, and further more preferably from 2 to 10 hours, from the viewpoint of industrial productivity.

Examples of the means for bringing the fatty acids into contact with glycerol include immersion, agitation, and passing liquids through a column packed with immobilized lipase using a pump or the like.

After the esterification reaction, a purification step usually used for fats or oils may be performed. Specific examples include steps of distillation, acid treatment, washing with water, decolorization, and deodorization.

As shown in the Examples described below, the administration of the fat or oil of the present invention to atopic dermatitis model mice suppressed the dermatitis score, which represents the degree of progression of atopic dermatitis, and auricular thickening, as well as the increase in transepidermal water loss (TEWL). In addition, an analysis of mRNA expression in the skin at the site of induced dermatitis showed that the increase in the expression of IL-4, which is a major Th2 cytokine, IL-1**β,** IL-6, TSLP, and IL-33, which are cytokines secreted by innate immune competent cells and keratinocytes, was suppressed in the group fed with the fat or oil of the present invention.

The above cytokines whose increased expression was suppressed by the fat or oil of the present invention have been reported to contribute to the inflammatory response and impaired barrier function in various inflammatory skin diseases including not only atopic dermatitis, but also seborrheic dermatitis, contact dermatitis, psoriasis, asteatotic eczema, and erythema nodosum (reference literatures: Adalsteinsson JA, Kaushik S, et al. Exp Dermatol. 2020 May;29(5):481-489; Lee HY, et al. Mediators Inflamm. 2013;2013:916497; Belge K, et al. F1000Prime Rep. 2014 Jan 2;6:4; Hanel KH, et al. Int J Mol Sci. 2013 Mar 26;14(4):6720-45; and Polycarpou A, et al. Front Immunol. 2017 Mar 13;8:233). Therefore, the fat or oil of the present invention can be an agent for preventing, treating, or improving inflammatory skin disease and an agent for improving skin barrier function (hereinafter also referred to as "agent for preventing, treating, or improving inflammatory skin disease and the like"), and can be used for preventing, treating, or improving inflammatory skin disease, for improving skin barrier function, as well as for producing an agent for preventing, treating, or improving inflammatory skin disease and the like.

Here, "use" can be in humans or non-human animals, and may be therapeutic or non-therapeutic. "Non-therapeutic" is a concept which does not include medical practice, i.e., does not include methods of operating on, treating, or diagnosing a human being, and more specifically, does not include methods by which a physician, a healthcare professional or person directed by a physician performs surgery, treatment, or diagnosis on a human being.

In the present specification, examples of the targeted "inflammatory skin diseases" include atopic dermatitis, seborrheic dermatitis, contact dermatitis, psoriasis, asteatotic eczema, erythema nodosum, neonatal acne/acneiform eruption, miliaria, folliculitis, infantile eczema, and dry skin. It is particularly suitable for atopic dermatitis. "Atopic dermatitis" refers to a chronic disease whose main lesion is pruritic eczema, with repeated exacerbations and remissions.

"Skin barrier function" means the function of preventing the evaporation of water from within the skin, and the function of preventing the entry of substances from the outside into the skin. In the present invention, it is suitable for improving the function of preventing evaporation of water from within the skin (water permeability barrier function). Improvement of the skin barrier function allows to treat or improve xerosis (asteatotic), asteatotic eczema, and the like, which are caused by impaired skin barrier function.

"Prevention" refers to the prevention or delay of the onset of a disease or symptom in an individual, or the reduction of the risk of onset of a disease or symptom in an individual.

"Improvement" refers to the amelioration of a disease, symptom, or condition, the prevention or delay of the aggravation of a disease, symptom, or condition, or the reversal, prevention, or delay of the progression of a disease or symptom.

Moreover, "treatment" also includes the improvement of symptoms in addition to the complete cure of the disease.

The agent for preventing, treating, or improving inflammatory skin disease and the like of the present invention can itself be a medicament, quasi-drug, cosmetic, food or feed for preventing, treating, or improving inflammatory skin disease, and for improving skin barrier function, or a material or preparation to be blended in the medicament, quasi-drug, cosmetic, food or feed.

The medicament (including quasi-drug, the same applies hereinafter) contains the fat or oil of the present invention as an active ingredient for preventing, treating, or improving inflammatory skin disease, and for improving skin barrier function. Furthermore, the medicament may contain a pharmaceutically acceptable carrier or other active ingredients, pharmacological ingredients, and the like, as needed, as long as the function of the active ingredient is not lost.

The medicament can be administered in any form of administration. Examples of forms of administration include oral solid preparations such as tablets (including chewable tablets), capsules, granules, powders, and lozenges; oral liquid preparations such as internal liquid medicines and syrups; and parenteral preparations such as injections, suppositories, inhalants, transdermal drugs, and topical products. The preferred form of administration is oral administration. The size of the form can be adjusted as desired according to the intended use.

These preparations of various dosage forms can be prepared in accordance with conventional methods by appropriately combining them with pharmaceutically acceptable carriers, such as excipients, binders, bulking agents, disintegrants, surfactants, lubricants, dispersants, buffers, osmotic pressure regulators, pH adjusters, emulsifiers, preservatives, stabilizers, preserving agents, thickeners, fluidity promoters, flavoring agents, foaming agents, flavors, coating agents, and diluents, or other pharmaceutical ingredients, as necessary.

The cosmetic contains the fat or oil of the present invention as an active ingredient for preventing or improving inflammatory skin disease, and for improving skin barrier function. Furthermore, the cosmetic may contain a cosmetically acceptable carrier or other active ingredients, pharmaceutical ingredients, cosmetic ingredients, and the like, as needed, as long as the function of the active ingredient is not lost.

Preferred examples of cosmetics containing the fat or oil of the present invention include cosmetics for the face and body (e.g., lotions, gels, creams, and facial masks), make-up cosmetics, and face or body cleansers.

Each of these cosmetic preparations can be produced in accordance with conventional methods. Examples of cosmetically acceptable carriers include various oils, surfactants, gelling agents, buffers, preservatives, antioxidants, solvents, dispersants, chelating agents, thickeners, ultraviolet absorbers, emulsion stabilizers, pH adjusters, pigments, and flavors.

Examples of other active, pharmaceutical, and cosmetic ingredients include plant extracts, germicides, moisturizing agents, anti-inflammatory agents, antibacterial agents, keratolytic agents, cooling agents, anti-seborrheic agents, cleansing agents, and makeup ingredients.

The food contains the fat or oil of the present invention as an active ingredient for preventing or improving inflammatory skin disease, and for improving skin barrier function.

The food includes those with claims of preventing or improving symptoms of inflammatory skin diseases, or of improving skin barrier function, and for which labeling to those effects have been approved or notified as necessary (food for specified health uses, food with functional claims, food for special dietary uses, and the like). Examples of labels include "helps retain moisture in the skin and alleviates dryness," and "improves skin discomfort." Foods for which functional claims are approved or notified can be distinguished from common foods.

The form of the food can be solid, semi-solid or liquid (e.g., beverage). Examples thereof include various food compositions (breads, cakes, noodles, confectioneries, frozen foods, ice cream, candy, furikake, soups, dairy products, shakes, beverages, seasonings, and the like), as well as nutritional supplement compositions in the same forms as the oral preparations described above (solid preparations such as granules, powders, tablets, capsules, microcapsules, and lozenges).

Foods in various forms can be prepared in accordance with conventional methods by appropriately combining the fat or oil of the present invention with food material, or other active ingredients, or food-acceptable additives (e.g., solvents, softeners, oils, emulsifiers, preservatives, acidulants, sweeteners, bittering agents, pH adjusters, stabilizers, coloring agents, ultraviolet absorbers, antioxidants, moisturizing agents, thickeners, fixing agents, dispersants, fluidity promoters, wetting agents, flavors, seasonings, and flavor modifiers) as desired.

The feed contains the fat or oil of the present invention as an active ingredient for preventing or improving inflammatory skin disease, and for improving skin barrier function.

The feed is preferably in the form of pellets, flakes, a mash or liquid, and examples thereof include livestock feed used for cattle, pigs, chickens, sheep, horses, and the like, feed for small animals used for rabbits, rats, mice, and the like, and pet food used for dogs, cats, small birds, and the like.

The feed can be prepared in accordance with conventional methods by appropriately combining the fat or oil of the present invention with other feed materials, such as meat, protein, grains, bran, sake lees, sugars, vegetables, vitamins, minerals, gelling agents, shape-retaining agents, pH adjusters, seasonings, preservatives, and nutritional enhancers.

The content of the fat or oil of the present invention in the preparation cannot be generalized as it varies depending on the form of the preparation, but for example, it is preferably 0.5 mass% or more, and preferably 99.8 mass% or less based on the total amount of the preparation.

Any dose or amount used of the fat or oil of the present invention can be used as long as it achieves the effects of the present invention. The dose or amount used can vary according to the subject' species, weight, sex, age, condition, or other factors, but in the case of oral administration (ingestion), it is, for example, from 0.3 to 15 g of the fat or oil of the present invention per adult (60 kg) per dose.

The above preparation can be administered or used according to any dosage regimen, and can be administered or used repeatedly and continuously for 1 day or more, preferably for 7 days or more, more preferably for 14 days or more, even more preferably for 30 days or more, in a single or a plurality of doses per day.

The agent for preventing, treating, or improving inflammatory skin disease and the like of the present invention is preferably administered or used in the form of a fat or oil composition.

If the agent for preventing, treating, or improving inflammatory skin disease and the like is in the form of a fat or oil composition, the content of the fat or oil of the present invention in the fat or oil composition is preferably 90 mass% or more, more preferably 95 mass% or more, and preferably 100 mass% or less, more preferably 99.9 mass% or less, from the viewpoint of usability.

The fat or oil composition preferably contains an antioxidant from the viewpoint of flavor, oxidation stability, suppression of coloration, and the like. The content of antioxidant in the fat or oil composition is preferably 0.005 mass% or more and 1.0 mass% or less, more preferably 0.04 mass% or more and 0.75 mass% or less, further more preferably 0.08 mass% or more and 0.5 mass% or less, from the viewpoint of flavor, oxidation stability, suppression of coloration, and the like.

The antioxidant is not particularly limited as long as it is used in foods, but is preferably at least one selected from the group consisting of natural antioxidants, lecithin, tocopherol, ascorbyl palmitate, ascorbyl stearate, dibutylhydroxytoluene (BHT) and butylhydroxyanisole (BHA).

Examples of the subjects to whom the agent for preventing, treating, or improving inflammatory skin disease and the like of the present invention is administered or used include humans who wish to prevent, treat, or improve inflammatory skin disease, preferably atopic dermatitis, and humans who wish to improve skin barrier function. Examples of non-human animals include non-human mammals such as anthropoid apes and other primates.

With respect to the embodiments described above, the present invention further discloses the following aspects.

<1> An agent for preventing, treating, or improving inflammatory skin disease comprising, as an active ingredient, a fat or oil in which a content of **α-**linolenic acid in fatty acids constituting the fat or oil is 40 mass% or more, and a content of diacylglycerol is 25 mass% or more.
<2> An agent for improving skin barrier function comprising, as an active ingredient, a fat or oil in which a content of **α**-linolenic acid in fatty acids constituting the fat or oil is 40 mass% or more, and a content of diacylglycerol is 25 mass% or more.
<3> A food for preventing or improving inflammatory skin disease comprising, as an active ingredient, a fat or oil in which a content of **α**-linolenic acid in fatty acids constituting the fat or oil is 40 mass% or more, and a content of diacylglycerol is 25 mass% or more.
<4> A food for improving skin barrier function comprising, as an active ingredient, a fat or oil in which a content of **α**-linolenic acid in fatty acids constituting the fat or oil is 40 mass% or more, and a content of diacylglycerol is 25 mass% or more.
<5> The agent or food according to any one of <1> to <4>, wherein the content of **α**-linolenic acid in the fatty acids constituting the fat or oil is preferably 45 mass% or more, more preferably 50 mass% or more, further more preferably 52 mass% or more, preferably 80 mass% or less, more preferably 70 mass% or less, further more preferably 60 mass% or less, and is 40 mass% or more and 80 mass% or less, preferably 45 mass% or more and 80 mass% or less, more preferably 50 mass% or more and 80 mass% or less, more preferably 50 mass% or more and 70 mass% or less, further more preferably 52 mass% or more and 70 mass% or less, further more preferably 52 mass% or more and 60 mass% or less.
<6> The agent or food according to any one of <1> to <5>, wherein a content of linoleic acid (C18:2) in the fatty acids constituting the fat or oil is preferably 5 mass% or more, more preferably 10 mass% or more, preferably 40 mass% or less, more preferably 30 mass% or less, further more preferably 20 mass% or less, and preferably 5 mass% or more and 40 mass% or less, more preferably 10 mass% or more and 30 mass% or less, further more preferably 10 mass% or more and 20 mass% or less.
<7> The agent or food according to any one of <1> to <6>, wherein a content of oleic acid (C18:1) in the fatty acids constituting the fat or oil is preferably 10 mass% or more, preferably 50 mass% or less, more preferably 40 mass% or less, further more preferably 30 mass% or less, and preferably 10 mass% or more and 50 mass% or less, more preferably 10 mass% or more and 40 mass% or less, further more preferably 10 mass% or more and 30 mass% or less.
<8> The agent or food according to any one of <1> to <7>, wherein a content of diacylglycerol in the fat or oil is preferably 30 mass% or more, more preferably 50 mass% or more, further more preferably 55 mass% or more, further more preferably 60 mass% or more, further more preferably 65 mass% or more, further more preferably 70 mass% or more, further more preferably 80 mass% or more, further more preferably 83 mass% or more, further more preferably 84 mass% or more, preferably 96 mass% or less, more preferably 95 mass% or less, further more preferably 94 mass% or less, and preferably 25 mass% or more and 96 mass% or less, more preferably 30 mass% or more and 96 mass% or less, further more preferably 50 mass% or more and 96 mass% or less, further more preferably 50 mass% or more and 95 mass% or less, further more preferably 55 mass% or more and 95 mass% or less, further more preferably 60 mass% or more and 95 mass% or less, further more preferably 65 mass% or more and 95 mass% or less, further more preferably 65 mass% or more and 94 mass% or less, further more preferably 70 mass% or more and 94 mass% or less, further more preferably 80 mass% or more and 94 mass% or less, further more preferably 83 mass% or more and 94 mass% or less, further more preferably 84 mass% or more and 94 mass% or less.
<9> The agent or food according to any one of <1> to <8>, wherein a content of **α**-linolenic acid in fatty acids constituting the diacylglycerol is preferably 40 mass% or more, more preferably 45 mass% or more, further more preferably 50 mass% or more, further more preferably 52 mass% or more, preferably 80 mass% or less, more preferably 70 mass% or less, further more preferably 60 mass% or less, and preferably 40 mass% or more and 80 mass% or less, more preferably 45 mass% or more and 80 mass% or less, further more preferably 50 mass% or more and 80 mass% or less, more preferably 50 mass% or more and 70 mass% or less, further more preferably 52 mass% or more and 70 mass% or less, further more preferably 52 mass% or more and 60 mass% or less.
<10> The agent or food according to any one of <1>, <3>, and <5> to <9>, wherein the inflammatory skin disease is preferably atopic dermatitis, seborrheic dermatitis, contact dermatitis, psoriasis, asteatotic eczema, erythema nodosum, neonatal acne/acneiform eruption, miliaria, folliculitis, infantile eczema, or dry skin, more preferably atopic dermatitis, seborrheic dermatitis, contact dermatitis, psoriasis, asteatotic eczema, or erythema nodosum, further more preferably atopic dermatitis.
<11> The agent or food according to any one of <2>, <4>, and <5> to <9>, wherein the skin barrier function is preferably water permeability barrier function.
<12> Use of a fat or oil for producing an agent for preventing, treating, or improving inflammatory skin disease, in which a content of α-linolenic acid in fatty acids constituting the fat or oil is 40 mass% or more, and a content of diacylglycerol is 25 mass% or more.
<13> A fat or oil for use in preventing, treating, or improving inflammatory skin disease, in which a content of α-linolenic acid in fatty acids constituting the fat or oil is 40 mass% or more, and a content of diacylglycerol is 25 mass% or more.
<14> Non-therapeutic use of a fat or oil for preventing, treating, or improving inflammatory skin disease, in which a content of **α**-linolenic acid in fatty acids constituting the fat or oil is 40 mass% or more, and a content of diacylglycerol is 25 mass% or more.
<15> The fat or oil or use according to any one of <12> to <14>, wherein the inflammatory skin disease is preferably atopic dermatitis, seborrheic dermatitis, contact dermatitis, psoriasis, asteatotic eczema, erythema nodosum, neonatal acne/acneiform eruption, miliaria, folliculitis, infantile eczema, or dry skin, more preferably atopic dermatitis, seborrheic dermatitis, contact dermatitis, psoriasis, asteatotic eczema, or erythema nodosum, further more preferably atopic dermatitis.
<16> Use of a fat or oil for producing an agent for improving skin barrier function, in which a content of **α-**linolenic acid in fatty acids constituting the fat or oil is 40 mass% or more, and a content of diacylglycerol is 25 mass% or more.
<17> A fat or oil for use in improving skin barrier function, in which a content of **α**-linolenic acid in fatty acids constituting the fat or oil is 40 mass% or more, and a content of diacylglycerol is 25 mass% or more.
<18> Non-therapeutic use of a fat or oil for improving skin barrier function, in which a content of **α**-linolenic acid in fatty acids constituting the fat or oil is 40 mass% or more, and a content of diacylglycerol is 25 mass% or more.
<19> The fat or oil or use according to any one of <16> to <18>, wherein the skin barrier function is preferably water permeability barrier function.
<20> The fat or oil or use according to any one of <12> to <19>, wherein a content of **α**-linolenic acid in fatty acids constituting diacylglycerol is 40 mass% or more.
<21> A method for preventing, treating, or improving inflammatory skin disease, comprising administering or applying, to a subject, a fat or oil in which a content of **α**-linolenic acid in fatty acids constituting the fat or oil is 40 mass% or more, and a content of diacylglycerol is 25 mass% or more.
<22> The method according to <21>, wherein the inflammatory skin disease is preferably atopic dermatitis, seborrheic dermatitis, contact dermatitis, psoriasis, asteatotic eczema, erythema nodosum, neonatal acne/acneiform eruption, miliaria, folliculitis, infantile eczema, or dry skin, more preferably atopic dermatitis, seborrheic dermatitis, contact dermatitis, psoriasis, asteatotic eczema, or erythema nodosum, further more preferably atopic dermatitis.
<23> A method for improving skin barrier function, comprising administering or applying, to a subject, a fat or oil in which a content of **α**-linolenic acid in fatty acids constituting the fat or oil is 40 mass% or more, and a content of diacylglycerol is 25 mass% or more.
<24> The method according to <23>, wherein the skin barrier function is preferably water permeability barrier function.
<25> The method according to any one of <21> to <24>, wherein a content of **α**-linolenic acid in fatty acids constituting diacylglycerol is 40 mass% or more.

### Examples

### Example 1

### [Preparation of fat or oil and specialty feed]

The fatty acids obtained by hydrolyzing linseed oil (manufactured by Archer Daniels Midland) with an enzyme were fractionated by cooling and centrifugation to obtain fractionated fatty acids. 300 parts by mass of the obtained fractionated fatty acid was mixed with 47 parts by mass of glycerol, and an esterification reaction was performed using a commercially available immobilized 1,3-regioselective lipase (Novozymes) as a catalyst. After filtration of the immobilized enzyme, the reaction product was molecularly distilled, treated with acid, and washed with water to obtain a treated oil. The treated oil was deodorized, and then an antioxidant was added to prepare a fat or oil containing diacylglycerol (DAG) derived from linseed oil (lin-DAG).

The linseed oil-derived DAG fat or oil (lin-DAG) prepared in accordance with the above method, linseed oil (lin-TAG, manufactured by Summit oil mill), and as a control, soybean oil (soy-TAG, manufactured by The Nisshin OilliO Group, Ltd.) were prepared. The same concentration of the same antioxidant as lin-DAG was also added to lin-TAG and soy-TAG.

Three types of specialty feed were prepared by adding 10 mass% of any of the above fats or oils to AIN-93G (Oriental Yeast Co., ltd.), which is used as mouse feed and from which the soybean oil (7 mass%) and cornstarch (3 mass%) originally contained were removed.

The glyceride and fatty acid compositions of lin-DAG, lin-TAG and soy-TAG are shown in Table 1. The C18:3 unsaturated fatty acid in the fatty acid composition of Table 1 is **α**-linolenic acid.

**[Table 1]**

| Analysis items | | | lin-DAG | lin-TAG | soy-TAG |
|---|---|---|---|---|---|
| Glyceride composition | MAG | [%] | 1.4 | 0.0 | 0.1 |
| | DAG | [%] | 84.3 | 2.5 | 1.4 |
| | TAG | [%] | 13.9 | 97.5 | 98.5 |
| Fatty acid composition | C14:0 | [%] | 0.0 | 0.1 | 0.1 |
| | C16:0 | [%] | 2.7 | 5.6 | 10.6 |
| | C16:1 | [%] | 0.1 | 0.1 | 0.1 |
| | C18:0 | [%] | 1.6 | 4.8 | 3.9 |
| | C18:1 | [%] | 23.2 | 19.9 | 24.8 |
| | C18:2 | [%] | 16.0 | 15.8 | 51.7 |
| | C18:3 | [%] | 54.8 | 52.5 | 7.1 |
| | C20:0 | [%] | 0.1 | 0.2 | 0.4 |
| | C20:5 | [%] | 0 | 0 | 0 |
| | C22:0 | [%] | 0.2 | 0.2 | 0.5 |
| | C22:6 | [%] | 0 | 0 | 0 |
| | Others | [%] | 1.2 | 1.0 | 0.9 |

The methods for analyzing glyceride and fatty acid compositions are as follows.

### (i) Glyceride composition of fat or oil

To a glass sample bottle were added about 10 mg of fat or oil sample and 0.5 mL of trimethylsilylating agent ("Silylating Agent TH", manufactured by Kanto Chemical). The bottle was sealed tightly, and heated at 70°C for 15 minutes. To this was added 1.0 mL of water and 1.5 mL of hexane, and the mixture was shaken. After leaving it to stand, the upper layer was subjected to gas chromatography (GLC) for analysis.

### <GLC Analysis Conditions>

### (Conditions)

Apparatus: Agilent 6890 series (manufactured by Agilent Technologies)
Integrator: ChemStation B 02.01 SR2 (manufactured by Agilent Technologies)
Column: DB-1ht 10 m x 0.25 mm x 0.2 µm (manufactured by Agilent J&W)
Carrier gas: 1.0 mL He/min
Injector: Split (1:50), T = 340°C
Injection volume: 1 µL
Detector: FID, T = 350°C
Oven temperature: Raised from 80°C to 340°C at 10°C/minute and held for 15 minutes

### (ii) Constituent fatty acid composition of fat or oil

Fatty acid methyl esters were prepared in accordance with the "Method for Preparation of Fatty Acid Methyl Esters (2.4.1.-1996)" in "Standard Methods for the Analysis of Fats, Oils, and Related Materials" edited by the Japan Oil Chemists' Society, and the obtained fat or oil samples were measured in accordance with the American Oil Chemists' Society Official Method Ce 1f-96 (GLC method).

### <GLC Analysis Conditions>

Column: CP-SIL88 50 m x 0.25 mm x 0.2 µm (VARIAN)
Carrier gas: 1.0 mL He/min
Injector: Split (1:50), T = 300°C
Injection volume: 1 µL
Detector: FID, T = 300°C
Oven temperature: 150°C held for 5 min → 1°C/min temperature increase → 160°C held for 5 min → 2°C/min temperature increase → 200°C held for 10 min → 10°C/min temperature increase → 220°C held for 5 min

The fatty acid compositions of diacylglycerol in lin-DAG, lin-TAG and soy-TAG are shown in Table 2.

**[Table 2]**

| Analysis items | | | lin-DAG | lin-TAG | soy-TAG |
|---|---|---|---|---|---|
| Fatty acid composition | C14:0 | [%] | 0 | 0 | 0 |
| | C16:0 | [%] | 2.5 | 6.9 | 13.4 |
| | C16:1 | [%] | 0.1 | 0 | 0 |
| | C18:0 | [%] | 1.4 | 4.8 | 5.5 |
| | C18:1 | [%] | 23.0 | 25.1 | 19.5 |
| | C18:2 | [%] | 16.0 | 21.7 | 50.5 |
| | C18:3 | [%] | 56.3 | 38.1 | 5.1 |
| | C20:0 | [%] | 0.1 | 0.5 | 0.9 |
| | C20:5 | [%] | 0 | 0 | 0 |
| | C22:0 | [%] | 0.1 | 0.8 | 1.3 |
| | C22:6 | [%] | 0 | 0 | 0 |
| | Others | [%] | 0.6 | 2.2 | 3.7 |

The method for analyzing the fatty acid composition of diacylglycerol is as follows.

The DAG fraction was extracted from each fat or oil sample and derivatized, then subjected to gas chromatography (GLC method) for analysis under the following conditions. In this instance, the measurement method was based on the American Oil Chemists' Society (AOCS) Official Method Ce 1f-96. The C18:3 unsaturated fatty acid in the fatty acid composition of Table 2 is **α-**linolenic acid.

### <GLC Analysis Conditions>

Apparatus: Agilent 7890B (manufactured by Agilent Technologies)
Column: CP-Sil 88 for FAME 50 m x 0.25 mm x 0.2 µm (manufactured by Agilent Technologies)
Carrier gas: 1.0 mL He/min
Injector: Split (1:50), T = 300°C
Injection volume: 1 µL
Detector: FID, T = 300°C
Oven temperature: 150°C held for 5 min → 1°C/min temperature increase → 160°C held for 5 min → 2°C/min temperature increase → 200°C held for 10 min → 10°C/min temperature increase → 220°C held for 5 min

### [Test Overview]

(1) Thirty-two NC/Nga male mice were divided into four groups: a non-sensitized group (soy-TAG (-) group) and transdermally sensitized groups (soy-TAG (+) group, lin-TAG (+) group and lin-DAG (+) group) (N=8 per group), and the mice in the transdermally sensitized groups were each fed with either of the above three specialty feeds ad libitum. Mice in the non-sensitized group were fed with the specialty feed containing soy-TAG ad libitum. After 30 days, 100 mg of Biostir AD ointment (Biostir Inc.) containing allergens derived from *Dermatophagoides farinae* was uniformly applied on the dorsal neck and auricular region of the mice in the transdermally sensitized groups. Four days after the first application of Biostir AD, 150 µL of 4 mass% sodium dodecyl sulfate (SDS) solution was uniformly applied on the dorsal neck and auricular region of the mice to induce rough skin, and then 100 mg of Biostir AD ointment was uniformly applied again to the same area. For the mice in the non-sensitized group, no Biostir AD ointment, but only the SDS solution was applied. This operation was performed four more times, at a frequency of twice a week, to induce atopic dermatitis. Twenty-two days after the first application of Biostir AD ointment, the mice were euthanized by cardiac blood collection under anesthesia by isoflurane inhalation, and skin samples were collected from the dorsal neck, which is the site of induced dermatitis. Before and 7, 14, and 21 days after the first application of Biostir AD ointment, the dermatitis score was visually assessed, auricular thickness was measured, transepidermal water loss (TEWL) was measured by a Tewameter (registered trademark) TM300 (Courage + Khazaka electronic GmbH), and mRNA expression of various cytokines in the skin at the site of induced dermatitis was analyzed.

The dermatitis score (AD score) was evaluated according to each of the indicators in Table 3 below. Each of the four indicators, i) redness/bleeding, (ii) crust formation/dryness, (iii) edema, and (iv) scratching/tissue loss, was evaluated (from 0 to 3 points each), and the total score obtained (from 0 to 12 points) was used as the final dermatitis score.

**[Table 3]**

| **Score** | **Redness/Bleeding** | **Crust formation/Dryness** | **Edema** | **Scratching/Tissue loss** |
|---|---|---|---|---|
| **0** | **No symptoms** | **No symptoms** | **No symptoms** | **No symptoms** |
| **1** | **Local redness on the back, but no bleeding associated with continuous scratching** | **Locally present on the back, slight whitening and desquamation of the skin** | **Slight thickening on either the left or right side** | **Non-continuous scratching on the auricles, but no loss of tissue** |
| **2** | **Sporadic redness on the back, but no bleeding associated with continuous scratching** | **Sporadically present on the back, or visible desquamation of the skin** | **Visible thickening and swelling on both auricles** | **Minor continuous scratching on the auricles, but no loss of tissue** |
| **3** | **Redness on the entire back, or bleeding associated with continuous scratching** | **Crusting on the entire back, or visible desquamation of the skin** | **Visible thickening, swelling, and deformation on both auricles, and feeling hard to the touch** | **Continuous scratching on the auricles, and loss of tissue** |

The mRNA expression in the skin at the site of induced dermatitis was analyzed according to the following.

Total RNA was extracted from the skin sampled from the dorsal neck using an RNeasy Mini kit (Qiagen) in accordance with the recommended protocol. cDNA was synthesized from the obtained total RNA using a SuperScript^{™} VILO^{™} cDNA Synthesis Kit (Thermo Fisher Scientific) in accordance with the recommended protocol. The obtained cDNA was then used to analyze the mRNA expression of various cytokines contained in the skin at the site of induced dermatitis by performing real-time PCR. In the real-time PCR, mRNA expression levels were detected by preparing samples using Taqman^{™} Fast Universal PCR Master Mix (2x) (Applied Biosystems) and Taqman^{™} probes specific to each gene. The Taqman^{™} probes used in the analysis of the expression of various cytokine genes are listed in Table 4 below. In addition, RPLPO was used as an endogenous control.

**[Table 4]**

| **Gene name** | **Assay ID** |
|---|---|
| **IL-4** | **Mm00445259_m1** |
| **IL-1β** | **Mm00434228_m1** |
| **IL-6** | **Mm00446190_m1** |
| **TSLP** | **Mm01157588_m1** |
| **IL-33** | **Mm00505403_m1** |
| **RPLPO** | **Mm00725448_s1** |

### (2) Results

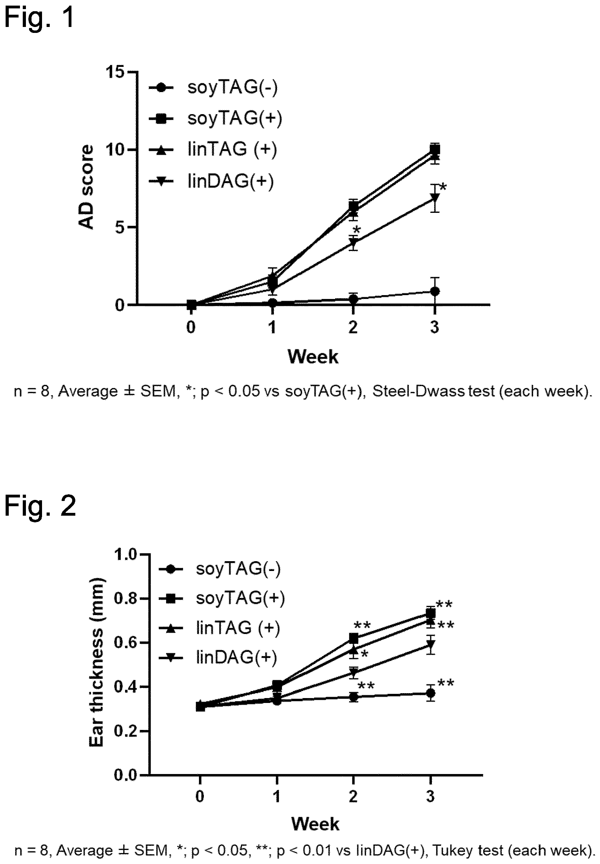

As shown in Figures 1, 2 and 3, compared to the non-sensitized soy-TAG (-) group, an increase in the dermatitis score, auricular thickening, and transepidermal water loss (TEWL) was observed in the transdermally sensitized soy-TAG (+) and lin-TAG (+) groups fed with the specialty feeds containing soy-TAG and lin-TAG, while the dermatitis score, auricular thickening, and TEWL were all significantly suppressed in the lin-DAG (+) group fed with the specialty feed containing lin-DAG.

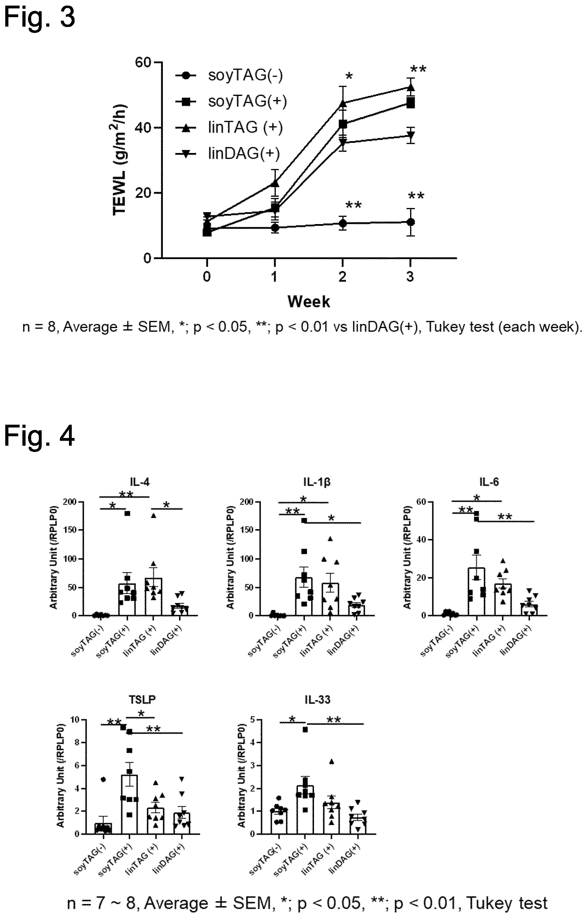

As shown in Figure 4, significant suppression of the increased expression of IL-4, IL-1β, IL-6, TSLP, and IL-33 was observed in the skin at the site of induced dermatitis in the lin-DAG (+) group.

These cytokines have been reported to contribute to the inflammatory response and impaired barrier function in various inflammatory skin diseases such as seborrheic dermatitis, contact dermatitis, psoriasis, asteatotic eczema, erythema nodosum, neonatal acne/acneiform eruption, miliaria, folliculitis, infantile eczema, and dry skin. Therefore, the fat or oil of the present invention can be considered useful in an agent for preventing, treating, or improving various inflammatory skin diseases including atopic dermatitis, seborrheic dermatitis, contact dermatitis, psoriasis, asteatotic eczema, erythema nodosum, neonatal acne/acneiform eruption, miliaria, folliculitis, infantile eczema, and dry skin.

## Claims

1. An agent for preventing, treating, or improving inflammatory skin disease comprising, as an active ingredient, a fat or oil in which a content of **α-**linolenic acid in fatty acids constituting the fat or oil is 40 mass% or more, and a content of diacylglycerol is 25 mass% or more.

2. The agent for preventing, treating, or improving inflammatory skin disease according to claim 1, wherein the inflammatory skin disease is atopic dermatitis, seborrheic dermatitis, contact dermatitis, psoriasis, asteatotic eczema, erythema nodosum, neonatal acne/acneiform eruption, miliaria, folliculitis, infantile eczema, or dry skin.

3. The agent for preventing, treating, or improving inflammatory skin disease according to claim 1 or 2, wherein a content of **α**-linolenic acid in fatty acids constituting diacylglycerol is 40 mass% or more.

4. An agent for improving skin barrier function comprising, as an active ingredient, a fat or oil in which a content of **α**-linolenic acid in fatty acids constituting the fat or oil is 40 mass% or more, and a content of diacylglycerol is 25 mass% or more.

5. The agent for improving skin barrier function according to claim 4, wherein the skin barrier function is water permeability barrier function.

6. The agent for improving skin barrier function according to claim 4 or 5, wherein a content of **α**-linolenic acid in fatty acids constituting diacylglycerol is 40 mass% or more.

7. A food for preventing or improving inflammatory skin disease comprising, as an active ingredient, a fat or oil in which a content of **α-**linolenic acid in fatty acids constituting the fat or oil is 40 mass% or more, and a content of diacylglycerol is 25 mass% or more.

8. The food for preventing or improving inflammatory skin disease according to claim 7, wherein the inflammatory skin disease is atopic dermatitis, seborrheic dermatitis, contact dermatitis, psoriasis, asteatotic eczema, erythema nodosum, neonatal acne/acneiform eruption, miliaria, folliculitis, infantile eczema, or dry skin.

9. The food for preventing or improving inflammatory skin disease according to claim 7 or 8, wherein a content of **α**-linolenic acid in fatty acids constituting diacylglycerol is 40 mass% or more.

10. A food for improving skin barrier function comprising, as an active ingredient, a fat or oil in which a content of **α**-linolenic acid in fatty acids constituting the fat or oil is 40 mass% or more, and a content of diacylglycerol is 25 mass% or more.

11. The food for improving skin barrier function according to claim 10, wherein the skin barrier function is water permeability barrier function.

12. The food for improving skin barrier function according to claim 10 or 11, wherein a content of **α**-linolenic acid in fatty acids constituting diacylglycerol is 40 mass% or more.

13. Use of a fat or oil for producing an agent for preventing, treating, or improving inflammatory skin disease, in which a content of **α**-linolenic acid in fatty acids constituting the fat or oil is 40 mass% or more, and a content of diacylglycerol is 25 mass% or more.

14. The use according to claim 13, wherein the inflammatory skin disease is atopic dermatitis, seborrheic dermatitis, contact dermatitis, psoriasis, asteatotic eczema, erythema nodosum, neonatal acne/acneiform eruption, miliaria, folliculitis, infantile eczema, or dry skin.

15. The use according to claim 13 or 14, wherein a content of **α**-linolenic acid in fatty acids constituting diacylglycerol is 40 mass% or more.

16. Use of a fat or oil for producing an agent for improving skin barrier function, in which a content of **α**-linolenic acid in fatty acids constituting the fat or oil is 40 mass% or more, and a content of diacylglycerol is 25 mass% or more.

17. The use according to claim 16, wherein the skin barrier function is water permeability barrier function.

18. The use according to claim 16 or 17, wherein a content of **α**-linolenic acid in fatty acids constituting diacylglycerol is 40 mass% or more.

19. A fat or oil for use in preventing, treating, or improving inflammatory skin disease, in which a content of **α**-linolenic acid in fatty acids constituting the fat or oil is 40 mass% or more, and a content of diacylglycerol is 25 mass% or more.

20. The fat or oil according to claim 19, wherein the inflammatory skin disease is atopic dermatitis, seborrheic dermatitis, contact dermatitis, psoriasis, asteatotic eczema, erythema nodosum, neonatal acne/acneiform eruption, miliaria, folliculitis, infantile eczema, or dry skin.

21. The fat or oil according to claim 19 or 20, wherein a content of **α**-linolenic acid in fatty acids constituting diacylglycerol is 40 mass% or more.

22. A fat or oil for use in improving skin barrier function, in which a content of **α**-linolenic acid in fatty acids constituting the fat or oil is 40 mass% or more, and a content of diacylglycerol is 25 mass% or more.

23. The fat or oil according to claim 22, wherein the skin barrier function is water permeability barrier function.

24. The fat or oil according to claim 22 or 23, wherein a content of **α**-linolenic acid in fatty acids constituting diacylglycerol is 40 mass% or more.

25. Non-therapeutic use of a fat or oil for preventing, treating, or improving inflammatory skin disease, in which a content of **α**-linolenic acid in fatty acids constituting the fat or oil is 40 mass% or more, and a content of diacylglycerol is 25 mass% or more.

26. The non-therapeutic use according to claim 25, wherein the inflammatory skin disease is atopic dermatitis, seborrheic dermatitis, contact dermatitis, psoriasis, asteatotic eczema, erythema nodosum, neonatal acne/acneiform eruption, miliaria, folliculitis, infantile eczema, or dry skin.

27. The non-therapeutic use according to claim 25 or 26, wherein a content of **α**-linolenic acid in fatty acids constituting diacylglycerol is 40 mass% or more.

28. Non-therapeutic use of a fat or oil for improving skin barrier function, in which a content of **α-**linolenic acid in fatty acids constituting the fat or oil is 40 mass% or more, and a content of diacylglycerol is 25 mass% or more.

29. The non-therapeutic use according to claim 28, wherein the skin barrier function is water permeability barrier function.

30. The non-therapeutic use according to claim 28 or 29, wherein a content of **α**-linolenic acid in fatty acids constituting diacylglycerol is 40 mass% or more.

31. A method for preventing, treating, or improving inflammatory skin disease, comprising administering or applying, to a subject, a fat or oil in which a content of **α**-linolenic acid in fatty acids constituting the fat or oil is 40 mass% or more, and a content of diacylglycerol is 25 mass% or more.

32. The method according to claim 31, wherein the inflammatory skin disease is atopic dermatitis, seborrheic dermatitis, contact dermatitis, psoriasis, asteatotic eczema, erythema nodosum, neonatal acne/acneiform eruption, miliaria, folliculitis, infantile eczema, or dry skin.

33. The method according to claim 31 or 32, wherein a content of **α**-linolenic acid in fatty acids constituting diacylglycerol is 40 mass% or more.

34. A method for improving skin barrier function, comprising administering or applying, to a subject, a fat or oil in which a content of **α**-linolenic acid in fatty acids constituting the fat or oil is 40 mass% or more, and a content of diacylglycerol is 25 mass% or more.

35. The method according to claim 34, wherein the skin barrier function is water permeability barrier function.

36. The method according to claim 34 or 35, wherein a content of **α**-linolenic acid in fatty acids constituting diacylglycerol is 40 mass% or more.
